# EUROPEAN PATENT SPECIFICATION

(11) **EP 3 677 234 B1**
(45) Date of publication and mention of the grant of the patent: **05.10.2022**
(21) Application number: 18850566.3
(22) Date of filing: 30.08.2018
(51) Int. Cl.: A61F 13/534, A61F 13/536, A61F 13/511

(54) **ABSORBENT ARTICLE**
ABSORBIERENDER ARTIKEL
ARTICLE ABSORBANT

(30) Priority: 30.08.2017 JP 2017165872
(43) Date of publication of application: 08.07.2020
(73) Proprietor: Daio Paper Corporation, Ehime 799-0492 (JP)
(72) Inventor: TAGOMORI, Junta, Sakura-shi Tochigi 329-1411 (JP)
(74) Representative: Epping - Hermann - Fischer
(86) International application number: PCT/JP2018/032106
(87) International publication number: WO 2019/044966

(56) References cited:
- EP-A1- 3 092 996
- EP-A1- 3 156 018
- WO-A1-2011/043256
- WO-A1-2013/099634
- WO-A1-2014/119601
- JP-A- 2005 087 655
- JP-A- 2013 154 016
- JP-A- 2017 104 263

## Description

### TECHNICAL FIELD

The present invention relates mainly to an absorbent article used for, for example, a sanitary napkin, a pantiliner, an incontinence pad, a medical pad, toiletries, and a disposable diaper, and more particularly relates to an absorbent article that includes a pulpless absorber and is configured such that embossments with good appearance can be formed.

### BACKGROUND ART

There exists a known absorbent article that is made by sandwiching a polymer sheet called a pulpless absorber including no pulp fiber between an impermeable back-side sheet such as a polyethylene sheet or a polyethylene-sheet-laminated nonwoven fabric and a permeable front-side sheet such as a nonwoven fabric or a permeable plastic sheet.

The absorbent article using the polymer sheet is advantageous in that the thickness of the absorber can be reduced and wearing discomfort can be reduced.

Also, there is a known technology for forming embossed grooves by heat embossing in a skin-side surface of the permeable front-side sheet to prevent diffusion of body fluid in the width direction and to prevent the absorber from being wrinkled and thereby improve the contact between the absorbent article and the skin.

For example, Patent Document 1 discloses an absorbent article in which embossed grooves are continuously formed along the longitudinal direction. Also, Patent Document 2 discloses an absorbent article in which a pair of first longitudinal compressed grooves and a pair of second longitudinal compressed grooves are continuously formed along the longitudinal direction. The second longitudinal compressed grooves are disposed on the outer sides of the first longitudinal compressed grooves in the width direction

### RELATED-ART DOCUMENTS

### Patent Documents

Patent Document 1: Japanese Laid-Open Patent Publication No. 2006-020976
Patent Document 2: Japanese Laid-Open Patent Publication No. 2015-093170
Patent document 3 : JP2013154016

### DISCLOSURE OF INVENTION

### PROBLEMS TO BE SOLVED BY THE INVENTION

In each absorbent article described in Patent Documents 1 and 2, the embossed grooves are formed continuously along the longitudinal direction of the absorbent article. Therefore, in a process of forming the embossed grooves, when the absorbent article is placed between an embossing roller and a flat roller and compressed sequentially from one end of the absorbent article in the longitudinal direction, stress and strain caused by the compression gradually accumulate as the length of the formed embossed grooves increases. When the formed embossed grooves reach a certain length, trapped stress and strain may cause the front-side sheet and the absorber to wrinkle and/or break and may damage the appearance of the embossed grooves. Document D3 relates to an absorbent article having a back surface sheet which comprises an edge part, wherein the edge part does not directly get in touch with the skin and thereby discomfort feeling is reduced when the absorbent article gets attached. D1 shows that the liquid permeable sheet is provided with embossments in the longitudinal direction, wherein the embossments are interrupted by non-compressed parts.

Particularly, in a pulpless absorbent article including, as a body-fluid absorber, a polymer sheet formed by placing a superabsorbent polymer between nonwoven sheets, the stress and strain caused by the compression of pulp fibers during the embossing are less likely to be relaxed unlike in an absorbent article including a relatively-thick absorber such as stacked fiber pulp. Accordingly, in the pulpless absorbent article, the front-side sheet and the absorber are more likely to be wrinkled and/or broken, and the appearance of the embossed grooves may be further damaged.

Also, there is a technology in which an embossed groove is formed like a discontinuous line by alternately arranging compressed parts and non-compressed parts. However, when the length of the non-compressed parts is short, the stress and strain caused when embossing the compressed parts cannot be sufficiently released at the non-compressed parts, and the front-side sheet and the absorber may be wrinkled and broken due to the stress and strain accumulated over a certain length, and the appearance of the embossed grooves may be damaged. Further, when the length of the non-compressed parts is too long compared to the length of the compressed parts, the body fluid tends to diffuse outward in the width direction through the non-compressed parts, and the absorbent article becomes less likely to deform along the embossments to suit the body shape. As a result, the function of the embossed grooves may be reduced, and the wearability of the absorbent article may be reduced.

Accordingly, the main object of the present invention is to provide an absorbent article that uses a polymer sheet as an absorber and is still configured such that embossments with good appearance can be formed and the function of the embossments can be reliably implemented.

### MEANS FOR SOLVING THE PROBLEMS

To solve the above problems, the invention of claim 1 provides an absorbent article that includes a polymer sheet including a superabsorbent polymer disposed between an upper-layer sheet disposed on a skin side and a lower-layer sheet disposed on a non-skin side, and an embossment that is formed on a skin-side surface of the polymer sheet along a longitudinal direction of the absorbent article and is shaped like a discontinuous line formed by arranging compressed parts and non-compressed parts alternately. The ratio A:B of a length A of each of the compressed parts to a length B of each of the non-compressed parts is between 7:1 and 1:1.

According to the invention of claim 1, in the absorbent article including the polymer sheet, the embossment is formed on the skin-side surface of the polymer sheet along the longitudinal direction of the absorbent article, and the embossment is shaped like a discontinuous line formed by arranging the compressed parts and the non-compressed parts alternately. Here, the ratio A:B of the length A of each of the compressed parts to the length B of each of the non-compressed parts is between 7:1 and 1:1. With this configuration, the ratio of the length B of the non-compressed part to the length A of the compressed part is constant. Therefore, the non-compressed part reliably functions as a relaxing part that releases stress and strain accumulated during the embossing process of the compressed part. This in turn makes it possible to prevent excessive accumulation of stress and strain and prevent the front-side sheet and the absorber from being wrinkled and/or broken, and thereby makes it possible to form an embossment with good appearance.

Also, because the length B of the non-compressed part is less than or equal to the length A of the compressed part, the effect of the embossment to block a body fluid is not significantly reduced, and the spread of the body fluid to the outside of the embossment in the width direction can be adequately suppressed. Also, when the absorbent article is worn, the absorbent article can be reliably deformed along the embossment so that the fit and the wearability of the absorbent article are improved. Thus, the function of the embossment is not degraded.

The invention of claim 2 provides the absorbent article according to claim 1 where the polymer sheet includes a middle sheet disposed between the upper-layer sheet and the lower-layer sheet, and the superabsorbent polymer is disposed between the upper-layer sheet and the middle sheet and between the middle sheet and the lower-layer sheet.

According to the invention of claim 2, the middle sheet is provided between the upper-layer sheet and the lower-layer sheet, and the superabsorbent polymer is disposed between each combination of the sheets. That is, the polymer sheet has a five-layer structure. Providing the middle sheet makes it possible to form a bulky polymer sheet and makes it easier to form a distinct embossment.

The invention of claim 3 provides the absorbent article according to claim 1 or 2 where the length of each of the compressed parts is between 3 mm and 70 mm.

According to the invention of claim 3, the length A of the compressed part is set at a value between 3 mm and 70 mm. If the length A of the compressed part is greater than 70 mm, the stress or strain accumulated during the embossing process becomes excessively large, and the front-side sheet and the absorber tend to be wrinkled and/or broken. On the other hand, if the length A of the compressed part is less than 3 mm, the bonding strength becomes too small. As a result, the embossment cannot be formed distinctly, and the appearance of the embossment is degraded.

The invention of claim 4 provides the absorbent article according to any one of claims 1 through 3 where the length of each of the non-compressed parts is between 0.4 mm and 50 mm.

According to the invention of claim 4, the length B of the non-compressed part is set at a value between 0.4 mm and 50 mm so that the stress or strain accumulated during the embossing process of the compressed part can be reliably released and the function of the embossment can be reliably implemented.

The invention of claim 5 provides the absorbent article according to any one of claims 1 through 4 where a groove width of the compressed parts is between 2 mm and 10 mm.

According to the invention of claim 5, the groove width of the compressed parts is set at a value between 2 mm and 10 mm. This configuration makes it possible to form a distinct embossment and to prevent the embossment from becoming hard and causing the wearer to feel discomfort.

### ADVANTAGEOUS EFFECT OF THE INVENTION

As described above, the present invention makes it possible to form embossments with good appearance even when a polymer sheet is used as an absorber and makes it possible to reliably implement the function of the embossments.

### BRIEF DESCRIPTION OF THE DRAWINGS

FIG. 1 is a partially cut-away view of a sanitary napkin 1 according to the present invention;
FIG. 2 is a cross-sectional view taken along line II-II of FIG. 1;
FIG. 3 is a cross-sectional view of a polymer sheet 4;
FIG. 4 is a cross-sectional view of a variation of the polymer sheet 4;
FIG. 5 is a plan view of the polymer sheet 4;
FIG. 6 is a cross-sectional view taken along line VI-VI of FIG. 5; and
FIG. 7 is an enlarged plan view of an embossment 20.

### DESCRIPTION OF EMBODIMENTS

Embodiments of the present invention are described below with reference to the accompanying drawings.

### (BASIC CONFIGURATION OF SANITARY NAPKIN 1)

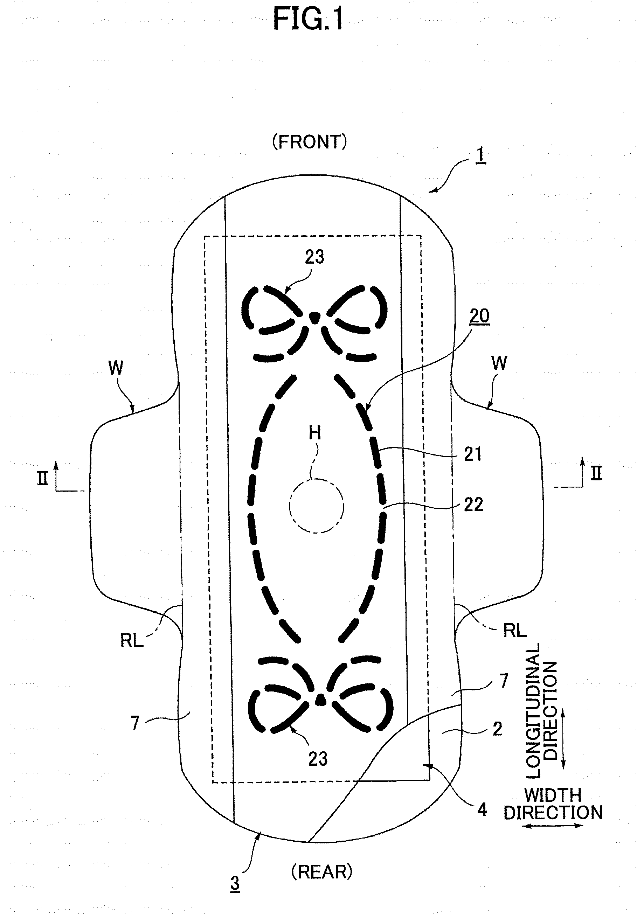

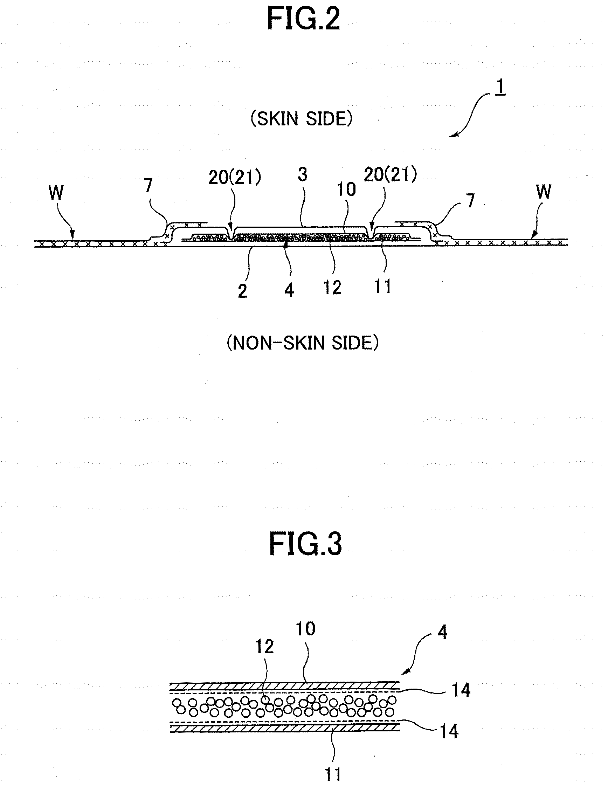

As illustrated in FIGs. 1 and 2, a sanitary napkin 1 of the present invention mainly includes an impermeable back-side sheet 2 formed of, for example, a polyethylene sheet; a permeable front-side sheet 3 that allows, for example, menstrual blood to quickly pass through; a polymer sheet 4 that is disposed between the sheets 2 and 3 and includes a superabsorbent polymer 12 disposed in a predetermined area between an upper-layer sheet 10 disposed on the skin side and a lower-layer sheet 11 disposed on the non-skin side; and side nonwoven fabrics 7 that are disposed on the sides of the skin-side surface and extend in the longitudinal direction. On the periphery of the polymer sheet 4, the outer edges of the impermeable back-side sheet 2 and the permeable front-side sheet 3 at the longitudinal ends of the polymer sheet 4 are bonded together with an adhesive such as a hot melt or by a bonding technique such as heat sealing or ultrasonic sealing. Also, at the side edges of the polymer sheet 4, parts of the impermeable back-side sheet 2 extending laterally beyond the polymer sheet 4 are bonded to the side nonwoven fabrics 7 with an adhesive such as a hot melt or by a bonding technique such as heat sealing or ultrasonic sealing. Also, as necessary, a hydrophilic second sheet (not shown) may be provided between the permeable front-side sheet 3 and the polymer sheet 4.

The structure of the sanitary napkin 1 is described in more detail below. The impermeable back-side sheet 2 may be formed of a sheet material such as polyethylene or polypropylene having at least a waterproof property. Also, a nonwoven fabric sheet that is made substantially impermeable by using a waterproof film (i.e., an impermeable back-side sheet including a waterproof film and a nonwoven fabric) may be used as the impermeable back-side sheet 2. In recent years, sheets having vapor permeability have been preferably used to prevent stuffiness. As a waterproof and vapor-permeable sheet, a microporous sheet is preferably used. The microporous sheet is obtained by melt-mixing an inorganic filler with an olefin resin such as polyethylene or polypropylene to form a sheet, and by stretching the sheet uniaxially or biaxially.

The permeable front-side sheet 3 is preferably made of a porous or nonporous nonwoven fabric or a porous plastic sheet. Examples of fiber materials of the nonwoven fabric include synthetic fibers formed of olefin such as polyethylene or polypropylene, polyester, or polyamide; regenerated fibers such as rayon and cupra, and natural fibers such as cotton. The nonwoven fabric may be produced by any appropriate production method such as spunlacing, spunbonding, thermal bonding, melt-blowing, or needle punching. Among these production methods, spunlacing has an advantage in terms of flexibility and draping characteristics, and thermal bonding has an advantage in terms of bulkiness and softness.

The side nonwoven fabrics 7 are provided on the sides of the skin-contacting surface of the sanitary napkin 1. The side nonwoven fabrics 7 extend in the longitudinal direction across almost the entire length of the sanitary napkin 1. Laterally-extending parts of the side nonwoven fabrics 7 and laterally-extending parts of the impermeable back-side sheet 2 form flaps, where the absorber 4 is not present, on the sides of the main body. Also, wing-shaped flaps W protruding outward are formed by laterally-extending parts of the side nonwoven fabrics 7 and laterally-extending parts of the impermeable back-side sheet 2. When the sanitary napkin 1 is worn, the wing-shaped flaps W are folded outward along folding lines RL (FIG. 1) at the base ends such that the wing-shaped flaps W are wound around the crotch of underwear. Anti-slip adhesive layers (not shown) are provided on the outer surface of the impermeable back-side sheet 2 corresponding to the wing-shaped flaps W and are attached to the outer surface of the underwear to prevent the slip between the sanitary napkin 1 and the underwear.

For the side nonwoven fabrics 7, a water-repellent nonwoven fabric or a hydrophilic nonwoven fabric may be used depending on the priorities of functions of the side nonwoven fabrics 7. For example, when priority is given to a function to prevent penetration of, for example, menstrual blood or discharge or to improve the feel, a water-repellent nonwoven fabric coated with a silicon-, paraffin-, or alkylchromic-chloride- repellent is preferably used. Alternatively, when priority is given to the absorbency of, for example, menstrual blood, a hydrophilic nonwoven fabric is preferably used. For example, such a hydrophilic nonwoven fabric may be prepared by polymerizing a compound having a hydrophilic group such as an oxidation product of polyethylene glycol during the production process of synthetic fibers. Also, a hydrophilic nonwoven fabric may be prepared by processing synthetic fibers with metal salt such as stannic chloride to partially dissolve the surfaces of the synthetic fibers and give them porosity, and by depositing metal hydroxide on the synthetic fibers. The resulting synthetic fibers become swollen or porous and exhibit a hydrophilic property due to capillarity. The side nonwoven fabrics 7 preferably include synthetic fibers so that the side nonwoven fabrics 7 can be fusion-bonded to the permeable front-side sheet 3.

### (POLYMER SHEET 4)

As illustrated in FIG. 3, the polymer sheet 4 disposed between the impermeable back-side sheet 2 and the permeable front-side sheet 3 has a structure where the superabsorbent polymer 12 is disposed between the upper-layer sheet 10 disposed on the skin side (on the side of the permeable front-side sheet 3) and the lower-layer sheet 11 disposed on the non-skin side (on the side of the impermeable back-side sheet 2), and is a so-called pulpless absorber that does not include pulp fibers. The superabsorbent polymer 12 is not dispersed in a fibrous material such as pulp between the upper-layer sheet 10 and the lower-layer sheet 11, but is present alone as a collection of particles of the superabsorbent polymer 12. This configuration makes it possible to reduce the thickness of the polymer sheet 4 and reduce the thickness of the sanitary napkin 1.

The upper-layer sheet 10 forming the skin-side layer of the polymer sheet 4 is implemented by a perforated or imperforate nonwoven fabric, crepe paper, a pulp sheet, or a porous plastic sheet. The nonwoven fabric is preferably formed of a hydrophobic fibrous material including chemical fibers. For example, similarly to the permeable front-side sheet 3, the nonwoven fabric may be formed of thermoplastic synthetic fibers that are formed of, for example, olefin such as polyethylene or polypropylene, polyester, or polyamide, or a mixture of these synthetic fibers and regenerated fibers such as rayon or cupra and/or natural fibers such as cotton. The nonwoven fabric may be produced by any appropriate method. However, to make the nonwoven fabric less likely to retain fluid, it is preferable to use a production method such as an air-through method with which the fiber density of an obtained product becomes low. Also, to prevent the superabsorbent polymer 12 from dropping off, a production method such as spunbonding, melt-blowing, or needle punching, with which the fiber density of an obtained product becomes high, may instead be used. Including chemical fibers in the upper-layer sheet 10 reduces the fluid retention of the upper-layer sheet 10, improves the fluid permeability of the upper-layer sheet 10 toward the lower layer, and reduces the chance of occurrence of a phenomenon where a body fluid is retained in the upper-layer sheet 10 and flows back toward the skin. To prevent the superabsorbent polymer 12 from dropping off, the pore diameter of the porous plastic sheet is preferably less than the average particle diameter of the superabsorbent polymer 12.

The mass per unit area of the upper-layer sheet 10 may be between 15 and 40 g/m² and preferably between 18 and 30 g/m², and the thickness of the upper-layer sheet 10 may be between 0.05 and 0.4 mm and preferably between 0.14 and 0.23 mm. The mass per unit area (g/m²) is measured by measuring the weight of a sample that is cut into a 20 mm × 40 mm (±2 mm) size with a roll cutter, and by converting the weight into a weight per 1 m². The thickness is measured using a thickness gauge (PEACOCK, large-size dial thickness gauge, model J-B (measurement range: 0 mm to 35 mm)) of OZAKI MFG. CO., LTD with the sample and the thickness gauge placed horizontally. Hereafter, the same methods may be used to measure mass per unit area and thickness.

The lower-layer sheet 11 forming the non-skin side layer of the polymer sheet 4 may be implemented by a perforated or imperforate nonwoven fabric, crepe paper, a pulp sheet, or a porous plastic sheet as well as by a waterproof sheet material. Similarly to the upper-layer sheet 10, the nonwoven fabric may be produced by any appropriate production method such as an air-through method, spunbonding, melt-blowing, or needle punching. To prevent the superabsorbent polymer 12 from dropping off, the pore diameter of the porous plastic sheet is preferably less than the average particle diameter of the superabsorbent polymer 12. As the waterproof sheet material, a material similar to the impermeable back-side sheet 2 may be used.

Examples of the superabsorbent polymer 12 include crosslinked polyacrylate, self-crosslinked polyacrylate, saponified product of crosslinked acrylate-vinyl acetate copolymer, crosslinked isobutylene-maleic anhydride copolymer, crosslinked polysulfonate, and partially-crosslinked water-swellable polymer such as polyethylene oxide or polyacrylamide. Among them, polymers including acrylic acid or acrylate are preferable in terms of the water absorption amount and the water absorption rate. The water absorbing power and the water absorption rate of the superabsorbent polymer having the water-absorbing capability can be adjusted by adjusting the crosslink density and the crosslink density gradient during the production process.

As illustrated in FIG. 3, the superabsorbent polymer 12 is preferably bonded to each of the upper-layer sheet 10 and the lower-layer sheet 11 via an adhesive layer 14 that is made of, for example, a hot melt adhesive and provided between the superabsorbent polymer 12 and each of the upper-layer sheet 10 and the lower-layer sheet 11.

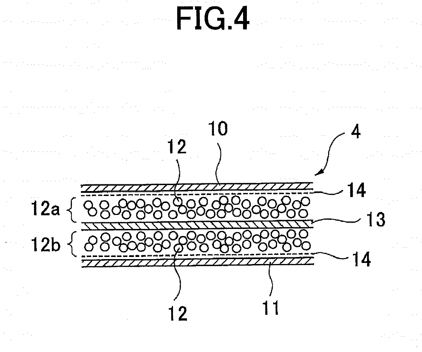

As a variation, as illustrated in FIG. 4, the polymer sheet 4 may have a five-layer structure where a middle sheet 13 is provided between the upper-layer sheet 10 and the lower-layer sheet 11, and superabsorbent polymers 12a and 12b are provided between the upper-layer sheet 10 and the middle sheet 13 and between the middle sheet 13 and the lower-layer sheet 11, respectively. With the structure where the middle sheet 13 is provided to divide the superabsorbent polymer layer into two layers, the upper superabsorbent polymer 12a and the lower superabsorbent polymer 12b are not appressed to each other even when the superabsorbent polymer absorbs water and swells, and therefore gel blocking can be suppressed. The middle sheet 13 may be implemented by a perforated or imperforate nonwoven fabric.

The polymer sheet 4 is preferably configured such that joints to join the upper-layer sheet 10 and the lower-layer sheet 11 are formed in a predetermined pattern to encapsulate the superabsorbent polymer 12 in predetermined areas between the upper-layer sheet 10 and the lower-layer sheet 11. That is, the polymer sheet 4 is preferably partitioned by the joints into one or more partitioned areas 17, and the superabsorbent polymer 12 is preferably placed in the partitioned areas 17.

The partitioned areas 17 may be formed in any pattern. For example, although not illustrated in figures, a joint may be formed along the periphery of the polymer sheet 4 such that one partitioned area 17 is formed across substantially the entire polymer sheet 4. Also, a grid-like joint may be formed in the one partitioned area 17 to form multiple partitioned areas 17 that are partitioned in a grid pattern.

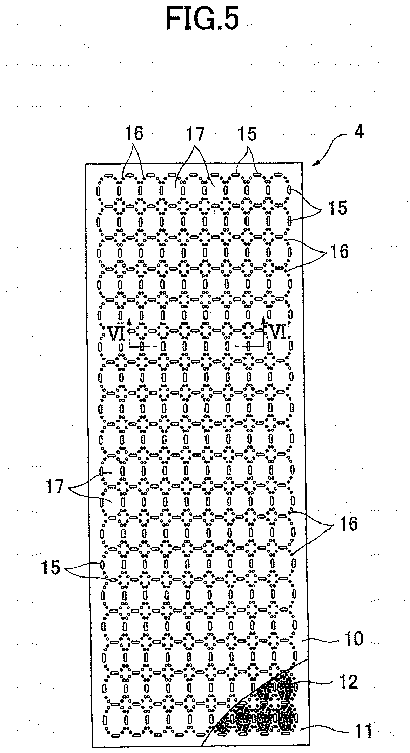

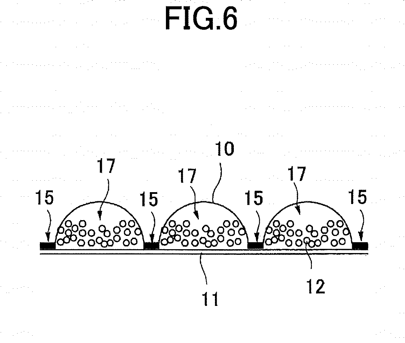

As a particularly preferable configuration, as illustrated in FIGs. 5 and 6, the polymer sheet 4 may be partitioned into substantially elliptical partitioned areas 17 by joints 15 and 16 joining the upper-layer sheet 10 and the lower-layer sheet 11, and the superabsorbent polymer 12 may be disposed in the partitioned areas 17. With this configuration, because the polymer arrangement area is partitioned into multiple partitioned areas 17, even when the superabsorbent polymer 12 encapsulated in each partitioned area 17 absorbs water and swells and the partitioned area 17 rises, the joints 15 and 16 surrounding the partitioned area 17 do not rise. Accordingly, a body fluid can smoothly spread through flow paths formed by the joints 15 and 16 even when the body fluid is repeatedly absorbed.

The planar arrangement pattern of the joints 15 and 16 illustrated in FIG. 5 are described in more detail. In plan view, the upper, lower, right, and left positions of each partitioned area 17 are surrounded by first joints 15 joining the upper-layer sheet 10 to the lower-layer sheet 11, and the oblique middle positions of the partitioned area 17 connecting the first joints 15 are surrounded by second joints 16 joining the upper-layer sheet 10 to the lower-layer sheet 11. Thus, the partitioned areas 17 are arranged in the longitudinal direction and the width direction of the sanitary napkin 1 in a positive grid pattern.

More specifically, in the polymer sheet 4, the upper-layer sheet 10 and the lower-layer sheet 11 are joined together by the first joints 15 arranged in a staggered grid pattern and are also joined together by the second joints 16 that are disposed in the oblique middle positions to connect the upper, lower, right, and left first joints 15 to each other. In the staggered grid pattern, adjacent rows or columns with the same pitch are arranged in alternate rows or alternate columns by shifting them by a half pitch such that the rows or the columns are arranged in alternate rows or alternate columns in the vertical direction and the horizontal direction. In the present application, the upper and lower positions indicate positions in a direction corresponding to the longitudinal direction (the front-rear direction) of the sanitary napkin 1, and the right and left positions indicate positions in a direction corresponding to the width direction of the sanitary napkin 1.

The superabsorbent polymer 12 is disposed in the partitioned areas 17 that are arranged in the longitudinal direction and the width direction of the napkin in a positive grid pattern in the polymer arrangement area and each of which is surrounded by the first joints 15 and the second joints 16.

Each of the first joints 15 disposed at the upper and lower ends of the partitioned area 17 is preferably shaped like a groove that is long in the horizontal direction (the width direction of the napkin), and each of the first joints 15 disposed at the right and left ends of the partitioned area 17 is preferably shaped like a groove that is long in the vertical direction (the longitudinal direction of the napkin). Thus, each first joint 15 is shaped like a groove that is long in the direction of a line connecting the first joint 15 to another first joint 15 of an adjacent partitioned area 17. This configuration facilitates the formation of a flow path that allows a body fluid to flow between adjacent partitioned areas 17. The first joint 15 is preferably shaped like a continuous groove. However, the first joint 15 may be shaped like intermittent dots.

The second joint 16 is preferably located apart from the first joint 15 and shaped like intermittent dots arranged in a direction to connect adjacent first joints 15. The second joint 16 may be shaped like intermittent dots with a bonding strength lower than that of the first joint 15 so that the second joint 16 is first separated when the superabsorbent polymer 12 absorbs fluid and swells, or may be shaped like a continuous groove.

The joints 15 and 16 are not formed continuously around the partitioned area 17 but are formed intermittently with unjoined portions between them. With this configuration, a body fluid flown into the partitioned area 17 can smoothly spread to an adjacent partitioned area 17 through the unjoined portions.

The joints between the upper-layer sheet 10 and the lower-layer sheet 11 may be formed by compressing the upper-layer sheet 10 and the lower-layer sheet 11 from the outer surface of the upper-layer sheet 10 and using thermal welding, ultrasonic welding, or a hot melt adhesive. When a hot melt adhesive is used, it is preferable that the adhesive is applied in a line shape or a plane shape using a slot, summit, or spiral application method, and particles of the superabsorbent polymer 12 are sprayed over the adhesive.

### (EMBOSSMENT 20)

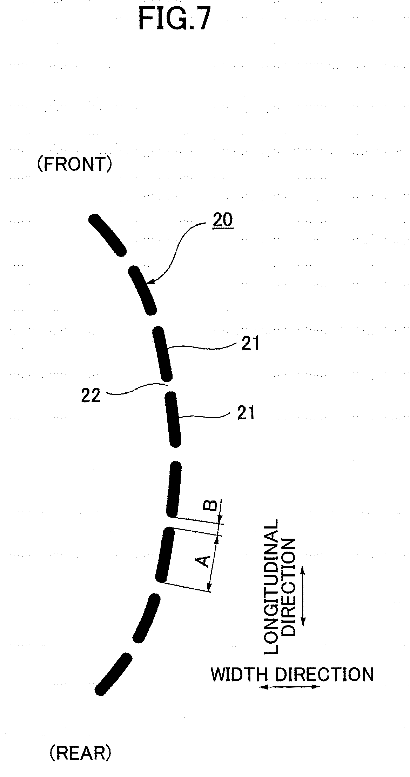

In the sanitary napkin 1, as illustrated in FIGs. 1 and 7, an embossment 20 is formed on the skin-side surface of the polymer sheet 4 along the longitudinal direction of the sanitary napkin 1. The embossment 20 is shaped like a discontinuous line formed by arranging compressed parts 21 and non-compressed parts 22 alternately.

The embossment 20 may be formed by indenting the components from the permeable front-side sheet 3 to the polymer sheet 4 together toward the non-skin side by pressing the skin-side surface of the permeable front-side sheet 3, or may be formed by indenting only the polymer sheet 4 toward the non-skin side by pressing the skin-side surface of the polymer sheet 4.

The compressed parts 21 are formed by indenting the polymer sheet 4 toward the non-skin side with a constant pressure. That is, each compressed part 21 does not include embossments, e.g., a high-compression part and a low-compression part, whose bottoms are at different depths, but the entire compressed part 21 has a constant depth. In the sanitary napkin 1, because the polymer sheet 4 is not as thick as a stacked fiber absorber, it is difficult to form a high-compression part and a low-compression part with a clear difference, and it may be difficult to form a distinct embossment 20.

The non-compressed part 22 is a portion that is not pressed from the skin side and is a gap provided between adjacent compressed parts 21. The non-compressed part 22 may be formed to have substantially the same height as the height of areas other than the embossment 20, or may be formed to be slightly indented toward the non-skin side due to the influence of the compressed parts 21.

Multiple compressed parts 21 and multiple non-compressed parts 22 are preferably formed along the longitudinal direction of the sanitary napkin 1. This ensures that the embossment 20 shaped like a discontinuous line has a desired function. The ratio of a length A of each compressed part 21 to a length B of each non-compressed part 22 (A:B) is between 7:1 and 1:1, preferably between 7:1 and 2:1, and more preferably between 6:1 and 4:1. Each of the lengths A and B is a length along the direction in which the embossment 20 extends. When the embossment 20 is shaped like a curve as illustrated in FIG. 7, each of the lengths A and B is a length (arc length) along the curve. The length B of the non-compressed part 22 may be a length along an extension line drawn from each of adjacent compressed parts 21 to the non-compressed part 22. Preferably, the multiple compressed parts 21 formed along the embossment 20 have substantially the same length across the entire embossment 20.

In the sanitary napkin 1, the ratio of the length B of the non-compressed part 22 to the length A of the compressed part 21 is constant. Therefore, the non-compressed part 22 reliably functions as a relaxing part that releases stress and strain accumulated during the embossing process of the compressed part 21. This in turn makes it possible to prevent excessive accumulation of stress and strain and prevent the permeable front-side sheet 3 and the polymer sheet 4 from being wrinkled and/or broken, and thereby makes it possible to form an embossment 20 with good appearance. When the length A of the compressed part 21 is seven times or more greater than the length B of the non-compressed part 22, the length of the compressed part 21 is too long, and stress or strain caused by the embossing process of the compressed part 21 tends to accumulate. When the accumulated stress or strain reaches a certain level, the permeable front-side sheet 3 and the upper-layer sheet 10 and the lower-layer sheet 11 of the polymer sheet 4 are wrinkled and/or broken. In terms of releasing the stress caused by the embossing process of the compressed part 21, the non-compressed part 22 may be formed longer than the compressed part 21. However, as described below, it is preferable to form the non-compressed part 22 at a certain ratio to secure the function of the embossment 20.

In the embossment 20, because the length B of the non-compressed part 22 is less than or equal to the length A of the compressed part 21, the effect of the embossment 20 to block a body fluid is not significantly reduced, and the spread of the body fluid to the outside of the embossment 20 in the width direction can be adequately suppressed. Also, when the sanitary napkin 1 is worn, the sanitary napkin 1 can be reliably deformed along the embossment 20 so that the fit and the wearability of the sanitary napkin 1 are improved. Thus, the function of the embossment 20 is not degraded. On the other hand, if the length B of the non-compressed part 22 is made longer than the length A of the non-compressed part 21, the amount of the body fluid spreading through the non-compressed part 22 to the outside of the embossment 20 in the width direction may increase, and the lateral leakage of the body fluid may occur. Also in this case, because the length B of the non-compressed part 22 is large relative to the length A of the compressed part 21, the portion of the napkin between adjacent compressed parts 21 does not readily deform along the embossment 20 and tends to bend at the non-compressed part 22 in an unintended direction. Accordingly, the napkin does not satisfactorily fit the shape of the body, and the wearability may be reduced. Particularly, because the polymer sheet 4 is used as an absorber in the sanitary napkin 1, if embossments extending in the longitudinal direction are formed as continuous compressed parts on the right and left sides of a body-fluid ejection part H, the outward spread of the body fluid in the longitudinal direction is blocked by the embossments, and gel blocking where the body fluid is retained near the body-fluid ejection part H tends to occur. However, because the embossment 20 according to the present invention is shaped like a discontinuous line formed by arranging the compressed parts 21 and the non-compressed parts 22 at a predetermined ratio, the body fluid can adequately spread to the outside of the embossment 20 in the width direction, and the gel blocking near the body-fluid ejection part H is less likely to occur.

Also, providing the non-compressed parts 22 makes it possible to intermittently stop the flow of the body fluid along the compressed parts 21 and thereby makes it possible to prevent the body fluid from excessively spreading along the embossment 20. On the other hand, if a compressed part is formed continuously without providing any non-compressed part, the body fluid tends to spread smoothly only in an area along the embossment. In this case, when a user sees a used sanitary napkin, the user may feel anxious about, for example, the leakage of the body fluid from edges of the absorber or whether the absorption capacity of the sanitary napkin is insufficient.

The length A of the compressed part 21 is preferably between 3 mm and 70 mm and more preferably between 5 mm and 20 mm. If the length A of the compressed part 21 is greater than 70 mm, the stress or strain accumulated during the embossing process becomes excessively large, and the permeable front-side sheet 3 and the polymer sheet 4 tend to be wrinkled and/or broken. On the other hand, if the length A of the compressed part 21 is less than 3 mm, the bonding strength of the embossment 20 becomes small. As a result, the embossment 20 cannot be formed distinctly and the appearance of the embossment is degraded.

The length B of the non-compressed part 22 is preferably between 0.4 mm and 50 mm and more preferably between 0.7 mm and 20 mm. If the length B of the non-compressed part 22 is less than 0.4 mm, the stress or strain accumulated when the compressed part 21 is compressed cannot be reliably released. If the length B of the non-compressed part 22 is greater than 50 mm, the function of the embossment 20 may be degraded. For example, the body fluid may spread easily through the non-compressed part 22.

The groove width (the width of the bottom of a groove) of the compressed part 21 is preferably between 2 mm and 10 mm and more preferably between 2 mm and 6 mm. This configuration makes it possible to form a distinct embossment 20 and to prevent the embossment 20 from becoming hard and causing the wearer to feel discomfort.

Each end of the compressed part 21 is preferably shaped like an arc that protrudes outward and is formed by connecting the ends of side walls of the compressed part 21. This configuration makes it possible to prevent the permeable front-side sheet 3 and the polymer sheet 4 from being broken at the ends of the compressed part 21. The arc is preferably a semicircular arc whose radius is one half of the groove width of the compressed part 21 (i.e., the diameter is the groove width).

Table 1 indicates the results of relative evaluation of the bonding strength, the appearance, and the hardness of the embossment 20 performed by changing the groove width of the compressed part 21. Table 2 indicates the results of relative evaluation of the bonding strength, the appearance, and the hardness of the embossment 20 performed by changing the length A of the compressed part 21. The evaluation results are indicated by ×: bad, ○: good, and ⊚: very good.

**[Table 1]**

| | Less than 1 mm | 1 mm | 2 mm | 5 mm |
|---|---|---|---|---|
| Bonding strength | × | × | ⊚ | ⊚ |
| Appearance | × | × | ⊚ | ⊚ |
| Hardness | ⊚ | ⊚ | ○ | ○ |

**[Table 2]**

| | Less than 1 mm | 5 mm | 10 mm | 20 mm |
|---|---|---|---|---|
| Bonding strength | × | O | ⊚ | ⊚ |
| Appearance | × | O | ⊚ | ⊚ |
| Hardness | ⊚ | ○ | ○ | ○ |

As illustrated in FIG. 1, one embossment 20 is preferably formed on each side of the body-fluid ejection part H such that a pair of right and left embossments 20 are formed. The embossments 20 are preferably formed in a predetermined longitudinal area including the body-fluid ejection part H. More specifically, the embossments 20 are formed in a longitudinal area that is longer than the length of the base end of each of the wing-shaped flaps W on the right and left sides. With this configuration, the embossments 20 can reliably block the body fluid spreading from the body-fluid ejection part H in width directions.

In the example illustrated in FIG. 1, each of the right and left embossments 20 is shaped like an arc that protrudes outward in the width direction. However, the embossment 20 may also be shaped like a straight line extending in the longitudinal direction of the sanitary napkin 1 or an arc protruding inward in the width direction as long as the embossment 20 is formed substantially along the longitudinal direction of the sanitary napkin 1.

Also, although one embossment 20 is formed on each side of the body-fluid ejection part H, two or more embossments 20 may be formed on each side of the body-fluid ejection part H. When multiple embossments 20 are formed on each side, the embossments 20 may be formed such that the compressed parts 21 and the non-compressed parts 22 in the adjacent embossments 20 are aligned with each other in the width direction of the sanitary napkin 1, or may be formed such that the compressed parts 21 and the non-compressed parts 22 are arranged alternately. The latter configuration can more effectively block the body fluid spreading outward in the width direction through the non-compressed parts 22 and is therefore more preferable.

The front ends and the rear ends of the right and left embossments 20 may be located apart from each other as illustrated in FIG. 1. Alternatively, although not illustrated, one or more embossments connecting the front ends and/or the rear ends to each other may be provided.

Also, a designed embossment(s) 23 may be provided in the middle in the width direction such that the designed embossment(s) 23 is disposed apart from the front end or the rear end of the right and left embossments 20. In the example of FIG. 1, the designed embossment 23 has a ribbon shape. However, the designed embossment 23 may have any other shape such as a heart shape or a star shape, or may be shaped in a geometric pattern such as a circle or a line. The designed embossment 23 may be shaped like a discontinuous line formed by arranging compressed parts and non-compressed parts alternately as in the embossment 20, or may be shaped like a continuous line formed by a continuous compressed part. The designed embossment 23 is preferably shaped like a discontinuous line to improve its appearance.

To form the embossments 20, a multilayer structure formed by stacking the permeable front-side sheet 3 on the skin-side surface of the polymer sheet 4 is placed between a flat roller and an embossing roller on which protrusions corresponding to the compressed parts 21 are formed, and the permeable front-side sheet 3 and the polymer sheet 4 are joined together by thermal pressure bonding at positions corresponding to the protrusions on the embossing roller.

### EXPLANATION OF REFERENCE NUMERALS

1 ... sanitary napkin, 2 ... impermeable back-side sheet, 3 ... permeable front-side sheet, 4 ... polymer sheet, 7 ... side nonwoven fabric, 10 ... upper-layer sheet, 11 ... lower-layer sheet, 12 ... superabsorbent polymer, 13 ... middle sheet, 14 ... adhesive layer, 20 ... embossment, 21 ... compressed part, 22 .... non-compressed part

## Claims

1. An absorbent article (1), comprising:
a polymer sheet (4) including a superabsorbent polymer (12) disposed between an upper-layer sheet (10) disposed on a skin side and a lower-layer sheet (11) disposed on a non-skin side; and
an embossment (20) that is formed on a skin-side surface of the polymer sheet (4) along a longitudinal direction of the absorbent article (1) and is shaped like a discontinuous line formed by arranging compressed parts (21) and non-compressed parts (22) alternately,
wherein a ratio A:B of a length A of each of the compressed parts (21) to a length B of each of the non-compressed parts (22) is between 7:1 and 1:1.

2. The absorbent article (1) as claimed in claim 1, wherein
the polymer sheet (4) includes a middle sheet (13) disposed between the upper-layer sheet (10) and the lower-layer sheet (11); and
the superabsorbent polymer (12) is disposed between the upper-layer sheet (10) and the middle sheet (13) and between the middle sheet (13) and the lower-layer sheet (11).

3. The absorbent article (1) as claimed in claim 1 or 2, wherein the length of each of the compressed parts (21) is between 3 mm and 70 mm.

4. The absorbent article (1) as claimed in any one of claims 1 through 3, wherein the length of each of the non-compressed parts (22) is between 0.4 mm and 50 mm.

5. The absorbent article (1) as claimed in any one of claims 1 through 4, wherein a groove width of the compressed parts (21) is between 2 mm and 10 mm.

## Patentansprüche

1. Absorbierender Artikel (1), aufweisend:
eine Polymerlage (4), die ein superabsorbierendes Polymer (12) enthält, das zwischen einer auf einer Hautseite vorgesehenen oberen Schichtlage (10) und einer auf einer Nicht-Hautseite vorgesehenen unteren Schichtlage (11) vorgesehen ist; und
eine Prägung (20), die auf einer hautseitigen Oberfläche der Polymerlage (4) entlang einer Längsrichtung des absorbierenden Artikels (1) gebildet ist und wie eine unterbrochene Linie geformt ist, die durch abwechselndes Anordnen von zusammengedrückten Teilen (21) und nicht zusammengedrückten Teilen (22) gebildet wird,
wobei ein Verhältnis A:B einer Länge A jedes der zusammengedrückten Teile (21) zu einer Länge B jedes der nicht zusammengedrückten Teile (22) zwischen 7:1 und 1:1 liegt.

2. Absorbierender Artikel (1) nach Anspruch 1, wobei
die Polymerlage (4) eine mittlere Lage (13) umfasst, die zwischen der oberen Schichtlage (10) und der unteren Schichtlage (11) vorgesehen ist; und
das superabsorbierende Polymer (12) zwischen der oberen Schichtlage (10) und der mittleren Lage (13) und zwischen der mittleren Lage (13) und der unteren Schichtlage (11) vorgesehen ist.

3. Absorbierender Artikel (1) nach Anspruch 1 oder 2, wobei die Länge jedes der zusammengedrückten Teile (21) zwischen 3 mm und 70 mm beträgt.

4. Absorbierender Artikel (1) nach einem der Ansprüche 1 bis 3, wobei die Länge jedes der nicht zusammengedrückten Teile (22) zwischen 0,4 mm und 50 mm beträgt.

5. Absorbierender Artikel (1) nach einem der Ansprüche 1 bis 4, wobei eine Rillenbreite der zusammengedrückten Teile (21) zwischen 2 mm und 10 mm beträgt.

## Revendications

1. Article absorbant (1), comprenant :
une feuille polymère (4) incluant un polymère superabsorbant (12) disposé entre une feuille de couche supérieure (10) disposée d'un côté peau et une feuille de couche inférieure (11) disposée d'un côté non peau ; et
un bossage (20) qui est formé sur une surface côté peau de la feuille polymère (4) le long d'une direction longitudinale de l'article absorbant (1) et est formé comme ligne discontinue formée en agençant en alternance des parties comprimées (21) et des parties non comprimées (22),
sachant qu'un rapport A:B d'une longueur A de chacune des parties comprimées (21) à une longueur B de chacune des parties non comprimées (22) est compris entre 7:1 et 1:1.

2. L'article absorbant (1) tel que revendiqué dans la revendication 1, sachant que
la feuille polymère (4) inclut une feuille médiane (13) disposée entre la feuille de couche supérieure (10) et la feuille de couche inférieure (11) ; et
le polymère superabsorbant (12) est disposé entre la feuille de couche supérieure (10) et la feuille médiane (13) et entre la feuille médiane (13) et la feuille de couche inférieure (11).

3. L'article absorbant (1) tel que revendiqué dans la revendication 1 ou 2, sachant que la longueur de chacune des parties comprimées (21) est comprise entre 3 mm et 70 mm.

4. L'article absorbant (1) tel que revendiqué dans l'une quelconque des revendications 1 à 3, sachant que la longueur de chacune des parties non comprimées (22) est comprise entre 0,4 mm et 50 mm.

5. L'article absorbant (1) tel que revendiqué dans l'une quelconque des revendications 1 à 4, sachant qu'une largeur de rainure des parties comprimées (21) est comprise entre 2 mm et 10 mm.
